Europäisches Patentamt

European Patent Office

Office européen des brevets

(19)

(11) Publication number : **0 450 936 A1**

# EUROPEAN PATENT APPLICATION

(12)

(21) Application number : 91302922.9

(22) Date of filing : 03.04.91

(51) Int. Cl.$^5$ : **C12P 21/08, C12N 5/18, A61K 39/395**

The applicant has filed a statement in accordance with Rule 28 (4) EPC (issue of a sample only to an expert). Accession number(s) of the deposit(s): ATCC HB - 10294

(30) Priority : 05.04.90 US 505191

(43) Date of publication of application :
09.10.91 Bulletin 91/41

(84) Designated Contracting States :
CH DE FR GB IT LI NL

(71) Applicant : MERCK & CO. INC.
126, East Lincoln Avenue P.O. Box 2000
Rahway New Jersey 07065-0900 (US)

(72) Inventor : Jacobson, Ethel B.
205 10th Street
Jersey City, NJ 07302 (US)
Inventor : Marburg, Stephen
50 Concord Avenue
Metuchen, NJ 08840 (US)

(74) Representative : Thompson, John Dr. et al
Merck & Co., Inc. European Patent
Department Terlings Park Eastwick Road
Harlow, Essex CM20 2QR (GB)

(54) Monoclonal antibody to avermectins.

(57) Novel monoclonal antibodies immunospecific for compounds of the avermectin complex are disclosed. A unique hybridoma cell line which produces the immunospecific monoclonal antibodies. The anti-avermectin monoclonal antibodies are used therapeutically to treat avermectin poisoning and serologically to identify and quantitate avermectins in animal body fluids.

EP 0 450 936 A1

## BACKGROUND OF THE INVENTION

The avermectins are a family of antiparasitic compounds which display activity against a broad range of internal and external parasites in animals. The avermectin complex, originally produced by fermentation of Streptomyces avermitilis MA-4680 (NRRL 8165), contains four closely related major components, $A_{1a}$, $A_{2a}$, $B_{1a}$, $B_{2a}$, in varying proportions and four minor components, $A_{1b}$, $A_{2b}$, $B_{1b}$, $B_{2b}$, each of which is a lower homolog of the corresponding major component. The structures of the natural components of the avermectin complex are described by Burg et al., Antimicrob. Agents and Chemother. 15: 361-367 ( 1979). Chemically modified derivatives of the natural avermectins are also well known in the art. The most widely used avermectin is 22, 23-dihydroavermectin $B_1$, known as ivermectin. Ivermectin consists of a major component, 22,23-dihydroavermectin $B_{1a}$ and a minor component of not more than 20% 22, 23-dihydroavermectin $B_{1b}$.

Ivermectin, and the entire complex of avermectins, act on susceptible nematodes and arthropods by potentiating the release and binding of gamma-aminobutyric acid (GABA) which sends inhibitory signals from interneurons to motoneurons. Signals from the CNS are therefore not perceived by the motoneurons and a state of flaccid paralysis of the parasite develops. Animals have a relative high tolerance for the avermectins presumably because the principle peripheral neurotransmitter is acetylcholine, which is not affected by the avermectins, and because they do not readily penetrate the CNS of animals where GABA functions as a neurotransmitter.

Even though the recommended doses of the avermectins offer a wide safety margin in livestock avermectin toxicity has been described. Avermectin toxicosis or poisoning has been reported for the following animal species; neonatal pigs, Can. Vet. J. 29: 735-736 (1988); kittens, Comp. Animal Pract. Sept. 32-32 (1988); dogs, J. Am. Vet. Med. Assoc. 191: 78-80 (1987); and calves, Aust. Vet. J. 65: 157-158 (1988). An acute toxic syndrome has been described for overdoses in various animals, Campbell and Benz J. Vet. Pharmacol. Therap. 7: 1-16 (1984). Animals generally show depression, ataxia and depending on the species and the level of overdose may result in death.

To date there has not been an effective antidote for avermectin poisoning described. Gamma-aminobutyric acid receptor antagonists, such as picrotoxin, have been proposed as antidotes to avermectin overdose. Nervous symptoms in dogs have been reduced with picrotoxin, Seman et al., Aust. Vet. J. 64: 284- (1987). Picrotoxin did not, however, reduce the symptoms of avermectin toxicity in calves, Button et al., Aust. Vet. J. 65: 157-158 (1988). The present invention allows the rapid and efficient treatment of avermectin toxicity by anti-avermectin antibody therapy.

The wide use of avermectins dictates that the levels of the drug in both animals which have been treated with avermectins and in humans which may have encountered the drug should be efficiently monitored. Avermectins in body fluids have generally been quantified by high performance liquid chromatography (HPLC), Alvinerie et al., Ann. de Res. Vet. 18: 269-274 ( 1987). Equivalent concentrations of avermectins in body fluids can be detected by the immunoassay of the present invention. This invention has the advantage of reducing the time necessary to determine the concentration of avermectins in body fluids by 1/3 when compared to the same determinations by HPLC.

## OBJECT OF THE INVENTION

It is, accordingly, an object of the present invention to provide hybridomas that secrete monoclonal antibodies that bind to natural and modified avermectins. Another object is to produce monoclonal antibody which reacts with avermectins to form an avermectin-antibody complex. A further object is to treat animals with avermectin toxicity or overdose with anti-avermectin antibody to reverse the toxicity. Another object is to allow the detection of avermectins in animal body fluids with an immunoassay which employs monoclonal anti-avermectin antibody.

## SUMMARY OF THE INVENTION

Novel monoclonal antibodies or antibody fragments immunospecific for compounds of the avermectin complex are disclosed. A unique hybridoma cell line which produces the immunospecific monoclonal antibodies. The anti-avermectin monoclonal antibodies are used therapeutically to treat avermectin poisoning and serologically to identify and quantitate avermectins in animal body fluids.

## DETAILED DESCRIPTION OF THE INVENTION

The present invention relates to the use of monospecific antibodies which bind to avermectins to detoxify

2

animals, including man. This monospecific antibody can also be used to detect the presence of and to quantify the amount of avermectin in animal body fluids, including man. Monospecific antibody as used herein is defined as a single antibody species or multiple antibody species with homogenous binding characteristics for the relevant avermectin epitope or antigen and immunologically reacts or binds to said epitope or antigen.

The avermectins, either the natural fermentation products or modifications thereof, are generally poor immunogens. Avermectin as used herein is defined as the naturally occurring avermectin complex and chemically modified derivatives of the natural avermectin complex which share a common antigen or epitope. In order to produce antibodies against common epitopes of the avermectin complex the immunogenicity of the epitope must be enhanced. Epitope is defined herein as an antigenic determinant site of known structure, in this case a portion of the avermectin which can react with preformed antibody and is present on many, if not all, members of the complex. Immunogens are prepared by attaching the common epitope or avermectin molecule onto an immunogenic carrier molecule capable of inducing antibody synthesis in animals. An immunogen is defined herein as a substance of sufficient size that when introduced into an animal, it stimulates the production of antibodies reactive with the specific antigen or epitope. Antigen is defined herein as a substance that can combine with a specific antibody. Immunogenic carrier is defined herein as a protein or other high molecular weight compound to which an antigen or epitope is conjugated in vitro and which renders the antigen or epitope capable of stimulating an immune response. The antigens of the present invention include, but are are not limited to, the common portions of the natural avermectins and those modified avermectins which have been chemically modified. The scope of antigen as defined herein also includes combinations of one or more of the avermectins. It is to be understood that the embodiment of this invention includes the use of the avermectins without the addition of immunologic carriers or any chemical modification, as direct stimulants of antibody formation under conditions that will permit the formation of monoclonal antibody.

Immunogens are prepared by covalently attaching any of the known avermectins, such as those described in Burg et al., Antimicrob. Agents and Chemother. 15: 361-367 (1979), or any chemically modified derivatives which exhibit antiparasitic activity to high molecular weight immunogenic carrier proteins resulting in avermectin protein conjugates. The preferred avermectin is avermectin $B_{1a}$. Carrier proteins include, but are not limited to bovine serum albumin (BSA), bovine thyroglobulin (BT), ovalbumin (OA), and the like, with BSA and BT being preferred. The avermectin-protein conjugates are prepared by reacting the protein which has been activated in an electrophilic sense with an avermectin derivatized to contain a nucleophilic amine. The proteins are succinylated and activated as p-nitrophenyl esters (Atassi, Biochemica et Biophysica Acta 670: 300 (1981), a method which allows the manipulation of these carrier proteins in either dimethylformamide (DMF) or dimethyl sulfoxide (DMSO ). This is necessary since the avermectins are very insoluble in aqueous systems. Bovine serum albumin and BT are succinylated by dissolving the protein in about 0.2 M sodium borate buffer, about pH 9.2 and then adding succinic anhydride dissolved in dioxane. The pH is maintained at about 9.2 by the simultaneous addition of NaOH. The succinylated BSA and BT are aged at about room temperature for about 2 hr and dialyzed three times against about 0.01 M triethylamine and two times against water. The dialysed material is lyophilized.

Succinylated BSA or BT, as prepared above, about 8.2 mg is dissolved in dimethylformamide (DMF) and p-nitrophenol is added to the solution, followed by dicyclohexyl carbodiimide in DMF. The mixture is stirred in the dark and 4"-(6-aminohexanoyl)-5-$^3$H avermectin $B_{1a}$ toluene sulfonic acid salt is added, followed by triethylamine. The mixture is stirred in the dark and separated on a Sephadex LH 60 column with DMF as the eluant. The fractions are dialyzed against water. The total dialysate contained about 1 µmole of tritiated avermectin as determined by specific activity and 3.9 mg of BSA as determined by Lowry analysis. The avermectin-BT solution contained a total of about 0.25 µmole of avermectin and about 6.8 mg protein. The avermectin-BSA complex is designated Avm-BSA while the avermectin-BT complex is designated Avm-BT.

Specific and monospecific antibodies to the avermectin immunogen(s) are purified from mammalian antisera containing antibodies reactive against the immunogen or prepared as monoclonal antibodies. Avermectin specific antibodies are raised by immunizing animals such as mice, rats, guinea pigs, rabbits, goats and horses, with rabbits and mice being preferred, with the specific immunogen. The animals may or may not receive booster injections following the initial immunization. At about 7 days after the initial immunization and after each booster immunization the animals are bled and the serum collected.

Anti-avermectin antibody secreting cells are obtained by immunizing animals, preferably mice, with the avermectin-carrier complex as described above. Female BALB/c mice are preferred. The mice receive a primary immunization by the intraperitoneal (IP) or subcutaneous (SC) route, with the IP route preferred, of about 5 µg to about 35 µg, preferably about 20 µg of Avm-BSA in an acceptable adjuvant. Acceptable adjuvants include, but are not limited to, complete Freund's (CFA), incomplete Freund's (IFA), alum-precipitate, water-in-oil emulsion containing Corynebacterium parvum and t-RNA, with CFA being preferred. Booster injections of about 10 µg Avm-BSA in phosphate buffered saline (PBS) are given at about 15, 60 and 90 days following the primary injection. The mice were rested for about 7 months and reimmunized. The animals initially received about 15 µg Avm-BSA in PBS, and about a week later were injected with about 20 µg in PBS. All mice received a final booster injection of about 40 µg of Avm-BSA in PBS about 6 months later. After about two additional months the animals are injected with about 75 µg Avm-BSA in PBS on three successive days. The injections are given IP, IV and IP. The spleens are removed about 1 day later. Antibody titers of the spleen cell donors are determined by the serological procedure described below.

Hybridoma cells are produced by mixing the anti-avermectin-$B_{1a}$ antibody producing splenic lymphocytes with an appropriate fusion partner under conditions which will allow the formation of stable hybridomas. Fusion partners may include mammalian myelomas such mouse, rat, rabbit or the like, with mouse being preferred. Murine fusion partners may include, but are not limited to, myeloma cell lines P3-NS1-Ag 4-1; MPC-11; S-194; NS.1 and Sp 2/0, with Sp 2/0 being preferred. The antibody producing cells and myeloma cells are fused in polyethylene glycol, about 1000 mol. wt., at concentrations of from about 30% to 60%, with 50% being preferred. Fusion is accomplished by mixing the antibody producing cells with the hypoxanthine-aminopterin-thymidine (HAT) sensitive myeloma cells at a ratio of about 4:1 in Dulbecco's Modified Eagles Medium (DMEM) and washing. Following centrifugation the cell pellet is resuspended in about 0.4 ml of a 50% solution of polyethylene glycol, 1000 mol. wt., and gently agitated for about 2 minutes. About 5 ml of DMEM is added dropwise to the cells and the cells are centrifuged at about 500 rpm for about 5 min and resuspended in DMEM containing about 10% fetal calf serum, $1 \times 10^{-4}$ M hypoxanthine, $1 \times 10^{-5}$ M thymidine and $8 \times 10^{-7}$ M aminopterin. The fused cells are plated at a density of about $1 \times 10^6$ cells per 1 ml per plate well. Hybridoma selection in the HAT medium is terminated after about 7 days, at which time fresh, normal (non-immune BALB/c) cells from the spleen or other lymphoid organs are added at a density of about $1 \times 10^5$ cells per well as feeder cells. The cultures are maintained in DMEM until anti-avermectin antibody is detected in the culture fluid. The presence of antibody may be detected by various serological or immunological assays such as precipitation, passive agglutination, enzyme-linked immunosorbent antibody (ELISA) technique, solid phase immunoradioassay (SPIRA) or liquid-phase radioimmunoassay, with a liquid phase assay being preferred. The antigen is tritiated avermectin-$B_{1a}$ ($^3$H-Avm) diluted to about 10 ng per ml in 0.05 M Tris buffer, pH 7.4, containing about 0.1% {3-[(3-cholamidopropyl)-dimethylammonio]-1-2-hydroxy-1-propanesulfonate}·$2H_2O$ (CHAPSO). About 50 µl of $^3$H-Avm (about 500 pg), about 100 µl of 0.1 % CHAPSO buffer and about 100µl of the test culture fluid are mixed and allowed to incubate at room temperature for about 2.5 hr. About 1 ml of activated charcoal (dilution predetermined) is added to each sample and most controls and the mixture was incubated for about 10 min at about 4° C. The charcoal is removed by centrifugation and the supernatant fluids collected and the amount of radioactivity determined in a Beckman scintillation counter. A total of 18 samples were positive, but only one designated 3A6 contained cells secreting anti-avermectin antibody. Antibody producing cells from 3A6 are cloned and sub-cloned by limiting dilution until a final clone designated 3A6-1 is obtained. The isotype of the monoclonal antibody produced by clone 3A6-1 is $IgG_1$ and is designated 3A6-1Mab. A sample of the hybridoma 3A6-1 was deposited in the American Type Culture Collection, 12301 Parklawn Drive, Rockville, Maryland 20852, USA, on November 7, 1989 under the Budapest Treaty and has been assigned ATCC number HB-10294.

The monoclonal antibody 3A6-1 Mab is specific for an epitope that is common to avermectin B1a, avermectin B2a and ivermectin and, thus appears common to all currently known avermectins. Specificity is determined by a competitive inhibition liquid phase RIA. The specificity is determined by contacting a known amount of labelled antigen ($^3$H-Avm $B_{1a}$) with constant amounts of other avermectins or ivermectin and then contacting this mixture with a constant amount of antibody specific for the labelled antigen. The concentration of antibody is an amount that is sufficient to bind about 50% of the labelled antigen employed in the assay. The results are shown in Table 1.

Monoclonal antibodies are produced in vivo by injecting pristane primed BALB/c mice approximately 0.5 ml per mouse, with about $2 \times 10^6$ 3A6-1 hybridoma cells about 4 days after priming. Acites fluid is collected at approximately 8-12 days and the monoclonal antibodies collected. The monoclonal antibody can be used as the acites fluid or the specific $IgG_1$ fraction can be separated or concentrated by precipitation. The antibody is precipitated with ammonium sulfate, about 35% to about 60% of saturation, with about 45% being preferred, washed and resuspended in a physiologically acceptable buffer at a pH of about 7.2. Such physiologically acceptable buffers include, but are not limited to, phosphate buffered saline, phosphate buffered saline glucose, buffered saline and the like.

The monoclonal antibody Mab 3A6-1 can effectively reverse the toxicity of a lethal dose of avermectin. The anti-toxic activity is effective both immediately after avermectin overdose and when the animals have been comatose for 24 hours or more. Animals, domestic or laboratory, preferably laboratory mice (BALB/c J, females) aged about 2 to 3 months are injected intraperitoneally with a lethal dose (about 12.5 mg/k) of avermectin $B_{1a}$ in sesame oil. Eighteen to 24 hours after avermectin injection, when the mice are fully comatose they are randomly divided into experimental (antibody treated) and control groups. Control groups receive either no antibody or injections with an unrelated mouse protein which correlates in concentration and composition to the antibody preparation being tested; i.e., crude Ascites or purified $IgG_1$. Ascites fluid containing Mab-3A6-1 and purified Mab-3A6-1 $IgG_1$ given to mice 18 to 24 hr after a toxic dose of avermectin is able to reverse the toxic effects of avermectin. The majority of the antibody treated animals exhibit signs of recovery within 4 to 7 hours after the first antibody injection and are completely recovered by the following day. The results are shown in Table 4-6.

Monoclonal antibody specifically reactive with a common epitope or common epitopes contained within the compounds of the avermectin family and derivatives or active fragment thereof are used to treat animals suffering from avermectin poisoning. A derivative or immunologically active fragment will include the F(ab')2, the Fab and any other fragment that can bind to epitope or epitopes of any of the avermectins. The invention is intended to include any fragments or derivatives of monoclonal antibodies to avermectins produced by recombinant antibody techniques such as those described, for example, by Lobuglio et al., Proc. Natl. Acad. Sci. USA 86: 4220-4224 (1989) or "humanized" animal monoclonals as disclosed in European Patent Publication No. 239,400 published September 30, 1987. It is also intended that the invention include any fractions of the variable portion of the heavy and light chains of the antiavermectin antibody, produced by recombinant technology, that can bind to the avermectin antigen. The monoclonal antibody or immunologically reactive fragments thereof will generally be administered to animals, including humans, as a parenteral suspension or solution with a physiologically acceptable medium. Such physiologically acceptable media include, but are not limited to, physiological saline, phosphate buffered saline, phosphate buffered saline glucose, buffered saline and the like. Parenteral administration includes inter alia, intramuscular, intraperitoneal, subcutaneous and intravenous injection or delivery of the monoclonal antibody. Avermectin, including ivermectin, toxicity is overcome by administration of from about 0.05 g to about 50 g of monoclonal antibody per kg body weight, with about 0.10 g to about 10 g monoclonal antibody per kg body weight being preferred.

Monoclonal antibody specifically reactive with a common epitope or common epitopes contained within the compounds of the avermectin family and derivatives thereof are used serologically to identify and quantitate avermectins, including ivermectin, in animal body fluids and cell-free extracts. Body fluids include, but are not limited to, blood, serum, plasma, milk, urine, cerebrospinal fluid and the like. Cell-free extracts as used herein are defined as the fluids derived from disrupted cells. The avermectins can be identified and the concentration determined by a variety of immunoassays or serological assays which include, but are not limited to: immunoradiometric assays, employing labelled antibodies, Miles and Hales, Nature 219: 186 (1986) or a sandwich variant according to Addison and Hales, Hormone. Meta. Res. 3: 59 (1971); enzyme immunoassays, employing competitive assays with antigen-enzyme conjugates according to the method of Van Weeman and Schours, F.E.B.S. Lett. 15: 232 (1971) or enzyme-labelled antibody assays according to the procedure of Envall and Perlman, Immunochem. 8: 871 (1971) or a variant of the enzyme-labelled antibody assay which employs detection by a second antibody according to the process of Hammarstrom et al., Proc. Natl. Acad. Sci. USA 72: 1528 (1975) or non-competitive sandwich (ELISA) methods, see Nakamura et al., in Handbood of Experimental Immunology, Vol. 1, Immunochemistry, D. M. Weir, ed. 4th Edition (1986), Blackwell Scientific Publications, Oxford, pp. 27.1-27.20; or any other assay which will determine the concentration or presence of avermectin. The preferred assay is a competitive inhibition liquid phase radioimmuno assay (RIA). The avermectin concentration in animal body fluids is determined by diluting the fluid in an appropriate assay buffer. An appropriate assay buffer may include, but is not limited to, buffered saline, phosphate buffer, phosphate buffered saline, Dulbecco's phosphate buffered saline, Dulbecco's calcium-and-magnesium-free buffered saline and Tris buffer containing about 0.1 % CHAPSO with Tris-CHAPSO being preferred. The labelled antigen ($^3$H-Avm) is diluted to a concentration of about 200 pg to about 700 pg, with about 500 pg being preferred, and contacted with an anti-avermectin monoclonal antibody at a concentration that will bind about 25% to about 75% of the labelled antigen, with about 50% being preferred. To the labelled antigen-antibody mixture is added an appropriate concentration or dilution of the unknown or sample containing avermectin or the standard containing a known amount of avermectin. The reactants are incubated at about 4° C to about 30° C, with about 25° C being preferred, for about 30 minutes to about 4 h, with about 2.5 h being preferred. All dilutions are made in the assay buffer and all determinations are made in duplicate. Non-antibody-bound radioactive avermectin is removed from the reaction mixture by chromatographic, precipitation, immunoprecipitation or adsorption techniques with adsorption of the unbound antigen onto dextran-coated activated charcoal being preferred. The dextran-coated charcoal is prepared by suspending activated charcoal, USP, at about 3% (w/v) in about 10 mM phosphate buffer, pH about 7.5, containing about 0.25% (w/v) dextran, about 60,000 to about 90,000 average mol. wt., with about 70,000 average mol. wt. being preferred. The dextran-coated activated charcoal is washed and resuspended in assay buffer and stored at a concentration of about 3%. About 1 ml of the dextran-coated activated charcoal suspension (dilution predetermined) is added to each sample and the samples are incubated for about 10 min at about 4° C. The charcoal is pelleted by centrifugation at about 3500 rpm for about 15 min. The supernatant quid is collected and the radioactivity counted in a liquid scintillation counter. Concomitant with the assay of fluid samples is the determination of controls of known avermectin concentration which produce values that are used to prepare a standard curve. The concentration of the unknown samples are then determined form the standard curve. This assay and the above noted equivalents allows the detection of avermectin in animal body fluids, see Tables 2 and 3.

The following examples illustrate the present invention without, however, limiting the same thereto.

EXAMPLE 1

Immunogen Preparation Avermectin $B_{1a}$ - Bovine Serum Albumin (Avm-BSA)

Succinylated BSA or BT was prepared by dissolving 100 mg of the protein in 20 ml of 0.20 M sodium borate buffer, pH 9.3 and then adding 550 mg of succinic anhydride dissolved in 2 ml of dioxane. The pH was maintained at 9.2 by the simultaneous addition of 2.5 N NaOH. The solution was aged at room temperature for 2 hr and dialyzed three times against 4 L of 0.01 M triethylamine and two times against 4 L of water. The succinylated proteins were lyophilized.

Succinylated BSA or BT, 8.2 mg, was dissolved in 0.7 ml of dimethylformamide (DMF) containing 3.2 mg p-nitrophenol to which was added 6.6 mg of carbodiimide in 0.4 ml of DMF. The BSA or BT mixture was maintained in the dark and stirred for 3.5 hr. Stirring was continued and 3.4 mg of 4"-(6-aminohexanoyl)-5-$^3$H avermectin $B_{1a}$ toluene sulfonic acid salt was added along with 50 µl of triethylamine. The mixture was stirred for an additional 22 h and then applied to a 55 ml Sephadex LH60 column (2.4 cm x 130 cm). Fractions, 1.3 ml, were collected following elution with DMF. The Avm-BSA and Avm-BT conjugates were in the first peak which was detectable by both u.v. and radioactivity. The trailing peak exhibited only radioactivity. The active fractions were dialyzed twice in Spectrapor 2 against 4 liters of water for 22 h and 44 h respectively. The total Avm-BSA dialysate contained 1 µ mole of tritiated avermectin by specific activity, and 3.9 mg of protein by Lowry analysis. The Avm-BT solution contained a total of 0.25 µmole of avermectin and 6.8 mg of protein.

EXAMPLE 2

Immunization Procedure

A ten week old female BALB/c J mouse, 10 weeks of age was injected intraperitoneally (IP) with 20 µg Avm-BSA, from Example 1, emulsified in complete Freund's adjuvant. Booster injections of 10 µg Avm-BSA in physiologic buffered saline (PBS) were given at 21, 60 and 90 days after the initial injection. The animal was rested for 7 months and reimmunized. The mouse was given 15 µg Avm-BSA in PBS and boosted with 20 µg Avm-BSA in PBS a week later and 40 µg Avm-BSA in PBS 6 months later. All injections were by the IP route. Anti-avermectin antibody production was confirmed from serum samples by serological procedures as described below. The animal was rested for two months and then given 75 µg of Avm-BSA in PBS on three successive days. The first injection was IP, while the second was intravenous and the third was IP.

Polyclonal antibody was prepared in rabbits. Three female New Zealand White rabbits were injected with 212 µ g of the Avm-BSA conjugate in Freund's complete adjuvant by the SC route. The animals were boosted with an equal amount in incomplete Freund's adjuvant by the same route two weeks later. The animals were bled one week after the first booster. After a 14 week rest period the rabbits were boosted a second time with 100 µg immunogen in PBS given SC and IV. One week later the rabbits received a third booster equivalent to the the second. All animals were bled one week later. A fourth booster, equal to the third, was given three weeks later and the animals were bled one week later. Serum was collected and anti-avermectin was obtained by standard methods.

EXAMPLE 3

Hybridoma And Monoclonal Antibody Production

Antibody producing cells were obtained from the spleens of animals producing anti-avermectin antibody, as described in Example 2. Spleen cells are isolated and purified by techniques known in the art. Hybridoma production was accomplished by mixing the anti-Avermectin-$B_{1a}$ antibody producing splenic lymphocytes with hypoxanthine-aminopterin-thymidine (HAT) sensitive murine myeloma cells, SP 2/0, at a cell ratio of 4:1 in Dulbecco's Modified Eagle's Medium (DMEM). Cell fusion was achieved by resuspending the cell pellet in 0.4 ml of a 50% solution of polyethylene glycol, 1000 mol wt, in DMEM and gently shaking for 2 minutes. Five ml of DMEM was added dropwise to the tube and the cells were centrifuged (500 rpm for 15 min) and resuspended in DMEM containing 10% fetal calf serum, $10^{-4}$ M hypoxanthine, $10^{-5}$ M thymidine and $8 \times 10^{-7}$ M aminopterin and plated in 24 well culture dishes at a density of $10^6$ spleen cells per ml per well. Hybridoma selection in the HAT medium was terminated after 7 days, at which time fresh, normal (non-immune) spleen cells were added at a density of $10^5$ cells per well as feeder cells, and the cultures were maintained in DMEM until anti-avermectin antibody was detected in the culture fluid, using a liquid-phase radioimmuno assay. Tritiated avermectin-B1a ($H^3$-Avm) was diluted to 10 ng per ml in 0.05 M Tris buffer, pH 7.4, containing 0.1% CHAPSO. Fifty μl of $^3$H-Avm (500 pg), 100 μl CHAPSO buffer and 100 μl of the test culture fluid were mixed thoroughly in 12 x 75 mm tubes and allowed to incubate at room temperature for 2.5 h. One ml of activated charcoal (dilution predetermined) was added to each tube and the tubes were incubated for 10 minutes at 4° C. The tubes were centrifuged at 4° C for 15 minutes at 3500 rpm to pellet the charcoal and the supernatant fluid was removed and added to vials containing 10 ml of scintillation fluid and counted in a Beckman scintillation counter. Of the 80 original wells plated from the single fusion only 18 were positive. Only 1 (3A6) of the 18 positive wells contained cells secreting specific anti-avermectin antibody. Antibody producing cells from this single well were cloned and then subcloned by limiting dilution procedures, after which the culture fluids were collected and assayed for antibody as described above. The final clone, designated 3A6-1, produced an anti-Avm monoclonal antibody characterized by double diffusion and ELISA as $IgG_1$.

The 3A6-1 monoclonal antibody (3A6-1mAb) was produced in in BALB/c mice. Hybridoma cells were grown in DMEM supplemented with 10% fetal calf serum to a cell density of approximately $1 \times 10^6$ cells per ml. The cells were collected, washed three times with DMEM and resuspended in DMEM at a concentration of $4 \times 10^6$ cells per ml. BALB/c mice were primed with pristane, 0.5 ml, intraperitoneally 4 days prior to the intraperitoneal injection of $2 \times 10^6$ hybridoma cells per animal. Ascites fluid was collected from the peritoneal cavity between 8 and 12 days after the hybridoma cell injection. The monoclonal antibody was partially purified by combining the ascites fluid with saturated ammonium sulfate to reach 45% of saturation. The precipitate was allowed to stand at 0° C for 2 h and then resolubilized in 0.1 M borate buffer, pH 8.2.

The monoclonal antibody 3A6-1 Mab was specific for an epitope that is common to avermectin B1a, avermectin B2a and ivermectin. Consequently, it is believed that the epitope is common to all currently known avermectins and the monoclonal antibody will react with all avermectins. Specificity is determined by a competitive inhibition liquid phase RIA. The specificity is determined by contacting a known amount of labelled antigen ($^3$H-Avm $B_{1a}$), 500 pg with constant amounts of other avermectins or ivermectin and then contacting this mixture with a constant amount of antibody specific for the labelled antigen. The concentration of antibody is an amount that is sufficient to bind about 50% of the labelled antigen employed in the assay. The results are shown in the following table.

## TABLE 1

## Monoclonal Antibody Specificity

| Inhibitor Inhibition | Concentration of inhibitor | % $^3$H-Tritium bound | % |
|---|---|---|---|
| a | - | 42 | - |
| $B_{1a}$ | 50 ng | 20 | 53 |
| $B_{2a}$ | 50 ng | 18 | 58 |
| Ivm | 50 ng | 16 | 63 |

a Mab alone

Inhibition is approximately the same for all three inhibitors indicating that 3A6-1 can recognize a determinant common to all three avermectins.

EXAMPLE 4

Detection Of Avermectin In Body Fluids

BALB/c mice were given intraperitoneal injections of avermectin $B_{1a}$, 10 mg per Kg in sesame oil. Blood was collected prior to injection and at 15, 30 and 60 minutes after the avermectin injection and the serum separated. The serum samples and the avermectin concentrations for the standard curve were assayed by the following procedure. Dilutions of body fluids and standard avermectin concentrations were prepared in CHAPSO assay buffer. The 3H-Avm was diluted in assay buffer to 500 pg per 0.05 ml and contacted with anti-avermectin antibody at a concentration sufficient to bind 50% of the labelled antigen. To this is added either a sample, containing an unknown amount of avermectin, or a standard, containing a known amount of avermectin, and the tubes are incubated at room temperature for 2.5 hours. The non-antibody-bound radioactive avermectin was removed by the addition of 1 ml of a suspension of dextran-coated activated charcoal (dilution predetermined). The charcoal was pelleted by centrifugation at 3500 rpm for 15 min. The supernatant fluid was collected and the radioactivity determined by liquid scintillation spectrophotometry. The four serum samples were tested at a 1:10 dilution in assay buffer and the values obtained were determined from a standard curve which was run simultaneously with the samples. The avermectin for the standard curve was prepared in 10-fold dilutions ranging from 50 ng to 0.5 ng. A standard curve was prepared by plotting the log of the avermectin concentration against the measured radioactivity. The avermectin concentration per tube of the sample was determined from the standard curve. The final values, concentration of avermectin per ml of serum, were obtained by correcting for the volume of inhibitor used (50 μl) and the dilution (1:10). The concentrations of avermectin in the mouse serum is given in the following table.

## TABLE 2

### Concentration Of Avermectin In Mouse Blood

| Time min | Concentration per tube | Concentration per ml serum |
|---|---|---|
| 15 | 2.4 ng | 480 ng |
| 30 | 13 ng | 2.6 µg |
| 60 | 42 ng | 8.4 µg |

The lowest value that can be determined with this curve, using undiluted serum, would be 10 ng/ml. The sensitivity could be increased by expanding the standard curve, increasing the amount of inhibitor used, or testing only undiluted serum.

Avermectin concentrations in milk were determined assaying samples of normal milk (control), milk containing 200 ng/ml avermectin (standard) and milk containing 50 ng/ml (test sample). The assay procedure is described above. The standard curve was prepared by assaying 2-fold dilutions of the standard, from 1:5 through 1:250 (5 ng to 0.04 ng per tube). The test sample was used at dilutions of 1:3, 1:6 and 1:9. The following table illustrates the ability to measure avermectin in milk using anti-avermectin antibody.

## TABLE 3

### Concentration Of Avermectin In Milk

| Dilution | Concentration per tube | Concentration per ml serum |
|---|---|---|
| 1:3 | 0.78 ng | 47 ng |
| 1:6 | 0.38 ng | 46 ng |
| 1:9 | 0.26 ng | 47 ng |

It should be noted that undiluted milk cannot be tested in this assay. Milk must be diluted at least 1:2 in EtOH before use in this assay.

These results show that avermectins and their derivitives can be identified in serum and milk samples. The described assays are sufficient for these determinations.

EXAMPLE 5

Reversal of Avermectin Toxicity

A total of 16 female BALB/c J mice, 2 to 3 months of age, were injected with a lethal dose of avermectin $B_{1a}$. The avermectin was dissolved in sesame oil and given as a single IP dose of 12.5 mg per Kg body weight. On the following day (18 to 24 h later), the mice were used for reversal studies. Of the 16 mice injected with avermectin, one mouse appeared unaffected and 1 mouse was dead. The remaining 14 mice, all of which were in deep coma, were randomly divided into 2 groups. The first group served as an uninjected control while the second group received a total of 3 ml of 3A6-1 ascites, containing 3A6-1 Mab, injected IP and SC over a period of 3.5 h. The results are shown in the following table.

## TABLE 4

### Effect of 3A6-1 Monoclonal Antibody on Avermectin Toxicity

| Treatment | Amount | Number | % Surviving |
|-----------|--------|--------|-------------|
| None | - | 7 | 0 |
| 3A6-1 Mab Ascites | 3.0 ml[a] | 7 | 86 |

[a] Contains 27 mg/ml total protein

Thirty female BALB/c J mice, 2 to 3 months of age were injected with avermectin as above. At 24 h post injection 1 mouse was unaffected and 7 mice were dead. The remaining 22 mice, all comatose, were divided into 3 groups. The first group received no treatment, the second group received CBPC-101 ascites fluid characterized as an $IgG_{2a}$ myloma protein with no known antibody specificity and third received 3A6-1 Mab ascites fluid. A total of 2.7 ml of the respective ascites fluid was injected over a period of 4.5 h. The results are shown in the following table.

## TABLE 5

### Effect of 3A6-1 Monoclonal Antibody on Avermectin Toxicity

| Treatment | Amount | Number | % Surviving |
|---|---|---|---|
| None | - | 7 | 0 |
| CBPC-101 Ascites | 2.7 ml[a] | 7 | 0 |
| 3A6-1 Mab Ascites | 2.7 ml[b] | 8 | 75 |

[a] IgG2 myeloma protein, 27 mg/ml total protein
[b] Contains 27mg/ml total protein

Thirty nine BALB/c J mice, 2 to 3 months of age were injected with avermectin as above. At 24 h post injection 1 mouse was unaffected, 1 mouse was dead and 2 mice were prostrate. The remaining 35 mice, all comatose, were divided into 3 groups. The first group received no treatment, the second group received MOPC-300 ascites fluid myloma protein which had been 2 x concentrated by ammonium sulfate precipitation and had an isotype of $IgG_1$, and the third group received 3A6-1 ascites Mab 2 x concentrated by ammonium sulfate precipitation. A total of 1.5 ml of the respective immunoglobulins were injected over a period of 5.5 h. The results are shown in the following table.

## TABLE 6

### Effect of Purified 3A6-1 Monoclonal Antibody on
### Avermectin Toxicity

| Treatment | Amount | Number | % Surviving |
|---|---|---|---|
| None | - | 11 | 36 |
| MOPC-300[a] | 1.5 ml | 12 | 8 |
| 3A6-1 Mab[b] | 1.5 ml | 12 | 100 |

[a] Purified IgG1, 9.5 mg/ml

[b] Partially purified containing 15 mg/ml protein, 7-10 of which is $IgG_1$ anti-Avm

These result clearly indicate that the toxic effect of avermectin can be reversed by treatment with anti-avermectin monoclonal antibody and more specifically by treatment with 3A6-1 Mab.

**Claims**

1. A monoclonal antibody or active fragment which is immunologically specific for one or more of the avermectins and their derivatives.

2. The monoclonal antibody of claim 1 wherein said monoclonal antibody is immunologically specific for avermectin $B_{1a}$, avermectin $B_{2a}$ and ivermectin.

3. The monoclonal antibody of claim 2 wherein the said monoclonal antibody is of the IgG1 isotype and is designated 3A6-1 Mab.

4. A hybridoma which produces monoclonal antibodies with said antibodies immunologically specific for avermectins and their derivatives.

5. The hybridoma of claim 4 wherein said hybridoma produces monoclonal antibodies which are immunologically specific for avermectin $B_{1a}$, avermectin $B_{2a}$ and ivermectin.

6. The hybridoma of claim 4 wherein the hybridoma is designated 3A6-1 and having the ATCC number HB-10294.

7. The method of producing a hybridoma of claim 1 which comprises the following steps:
   (1) immunizing mice with avermectin;
   (2) fusing the spleen cells from the immunized mice with mouse myeloma cells;
   (3) separating the hybrid cells from the non-fused cells;
   (4) selecting the hybrids which produce monoclonal antibodies that immunologically react with avermectin $B_{1a}$, avermectin $B_{2a}$ and ivermectin; and
   (5) isolating the monoclonal antibodies after the growth of the cells in vivo or in vitro.

8. The method of producing a hybridoma of claim 3 which comprises the following steps:
    (1) immunizing mice with avermectin;
    (2) fusing the spleen cells from the immunized mice with mouse myeloma cells;
    (3) separating the hybrid cells from the non-fused cells;
    (4) selecting the hybrids which produce monoclonal antibodies that immunologically react with avermectin $B_{1a}$, avermectin $B_{2a}$ and ivermectin; and
    (5) isolating the monoclonal antibody producing hybrids.

9. A pharmaceutical composition for reversing avermectin toxicity comprising an effective amount of monoclonal antibody or active fragment which is reactive with one or more avermectins and their derivatives in a pharmaceutical carrier.

10. The use of the pharmaceutical composition of claim 9 for the manufacture of a medicament for treating or preventing avermectin toxicity.

11. A method for detecting avermectins or avermectin derivatives in body fluids comprising:
    (1) contacting the avermectin containing body fluid with monoclonal antibody or reactive fragment which is specifically reactive with avermectin to form an avermectin-monoclonal antibody mixture;
    (2) measuring the extent of the reaction by serological or immunological assays and determining a value; then
    (3) comparing the values of the experimental samples with standard values.

12. An avermectin immunogen comprising one or more of the compounds of the avermectin group or derivatives thereof covalently attached to a carrier protein.

13. The avermectin immunogen of claim 12 wherein the avermectin is avermectin $B_{1a}$.

14. The avermectin immunogen of claim 12 wherein the carrier protein is bovine serum albumin.

15. The avermectin immunogen of claim 12 wherein the carrier protein is bovine thyroglobulin.

16. An antibody or active fragment thereof which is immunologically specific for one or more of the avermectins and their derivatives.

**European Patent Office**

**EUROPEAN SEARCH REPORT**

Application Number

EP 91 30 2922

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | No further relevant documents have been found. ----- | | C 12 P 21/08 C 12 N 5/18 A 61 K 39/395 |

TECHNICAL FIELDS SEARCHED (Int. Cl.5)

C 07 K
C 12 P

The present search report has been drawn up for all claims

| Place of search | Date of completion of the search | Examiner |
|---|---|---|
| THE HAGUE | 09-07-1991 | REMPP G.L.E. |